# EUROPEAN PATENT APPLICATION

(11) **EP 1 842 540 A1**
(43) Date of publication of application: **10.10.2007**
(21) Application number: 05844845.7
(22) Date of filing: 28.12.2005
(51) Int. Cl.: A61K 31/36, A23L 1/30, A61K 47/22, A61P 1/16, A61P 3/06, A61P 9/12, A61P 39/06, A61P 43/00

(54) **SESAMIN/EPISESAMIN COMPOSITIONS**

(30) Priority: 28.12.2004 JP 2004381726; 28.12.2004 JP 2004381806
(71) Applicant: SUNTORY LIMITED, Osaka-shi, Osaka 530-8203 (JP)
(72) Inventor: ONO, Yoshiko, Osaka-shi, Osaka 5330022 (JP); TOMIMORI, Namino, Kyoto-shi, Kyoto 6101145 (JP); TOKUDA, Saki, 6170833 (JP); ROGI, Tomohiro, Mishima-gun, Osaka 6180001 (JP)
(74) Representative: Vossius & Partner
(86) International application number: PCT/JP2005/024028
(87) International publication number: WO 2006/070856

(57) **Abstract**

Sesamin and episesamin are incorporated at specified weight ratios in oral compositions so that the oral absorption of episesamin and/or its transfer into blood circulation is increased. Sesamin and episesamin are also incorporated at specified weight ratios in oral compositions so that the physiological activities of sesamins are enhanced.

## Description

### FIELD OF THE INVENTION

This invention relates to sesamin and episesamin-containing compositions (sesamin/episesamin compositions) in which both sesamin and episesamin which is an epimer (isomer) thereof are contained in specified proportions, thereby enhancing the physiological activities of sesamins.

The invention further relates to foods and beverages, liquid beverages, pharmaceutical compositions, animal feed and pet food that have the sesamin/episesamin compositions added thereto or contained therein.

### BACKGROUND ART

The term "sesamins" as used hereinafter collectively refers to sesamin and episesamin. A mixture of sesamin and episesamin is hereinafter designated a sesamin/episesamin composition.
Episesamin is a stereoisomer of sesamin. To be more specific, sesamin is an optically active compound having the structure represented by formula I:

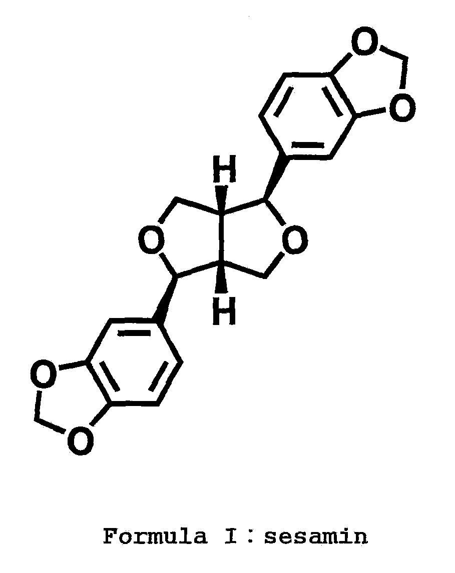

and episesamin, an isomer of sesamin, is an optically active compound having the structure represented by formula II:

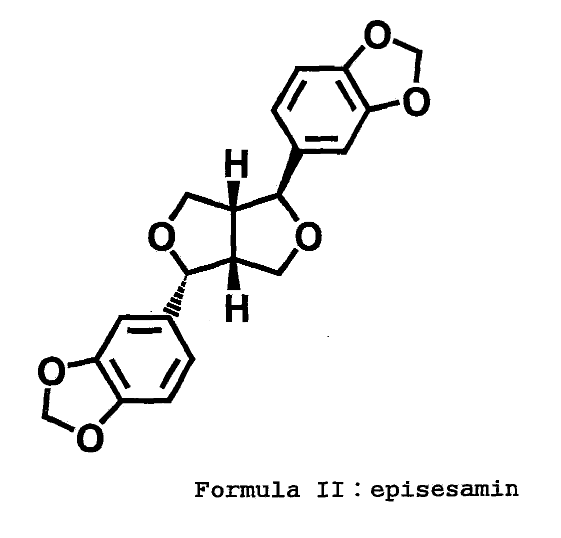

As is clear from the two formulas, sesamin has a symmetrical structure in the plane whereas episesamin has an asymmetric structure.

Sesamin is one of the principal lignan compounds in sesame and is contained in sesame seeds in amounts of 0.1-0.5%. In contrast, episesamin does not naturally occur in sesame seeds but when sesame oil passes through the steps of purification (decoloration and deodorization) to salad oil and the like, sesamin undergoes epimerization to give episesamin as a by-product (Namiki et al., "Goma -- Sono Kagaku to Kinousei (Sesame -- Its Science and Functions)", Maruzen Planet Co., Ltd. (1998)), and sesamins refined from sesame oil are known to contain sesamin and episesamin in proportions of nearly 1:1 by weight ratio (Fukuda, Y. et al., J. Am. Oil Chem. Soc., 63, 1027-1031 (1986)).
Prior art references disclose the following physiological activities of sesamins. For example, experiments with refined sesamins have revealed the following actions: inhibiting the metabolism of cholesterol or bile acids in the intestines (Japanese Patent No. 3183664); alleviating alcoholism and symptoms of withdrawal (USP 4,427,694); improving hepatic functions (Japanese Patent No. 3075358); in vivo stabilization of highly unsaturated aliphatic acids (JP 11-269456 A); inhibiting Δ5-unsauration enzymes (S. Shimizu et al., J. Am. Oil Chem. Soc., 66, 237-241 (1989), S. Shimizu et al., Lipid, 26, 512 (1991), and Japanese Patent No. 3070611); suppressing migraine (JP 2003-183172 A); inducing apoptosis in human leukemic cells (JP 2001-151676 A); and suppressing the oxidative decomposition of melatonin (JP 2000-143546 A). The action of inhibiting Δ5-unsaurating enzymes reported in Japanese Patent No. 3070611 and the action of inducing apoptosis in human leukemic cells reported in JP 2001-151676 A have been verified not only in refined sesamin but also in episesamin.
Experiments with sesame oil containing sesamin have revealed the action of alleviating inflammatory disease (amyotrophic lateral sclerosis) (JP 2005-533042 A), antiinflammatory and phylactic actions (JP 10-500937 A) and the allergy preventing or alleviating action of combination with fat or oil containing at least one of α-linolenic acid, eicosapentaenoic acid and docosahexaenoic acid (JP 5-58902 A).
In addition, the following reports have been published concerning sesamin/episesamin compositions. For example, experiments with sesame oil extracts containing lignan compounds as a main ingredient (19.6% sesamin, 30.6% episesamin, 10.2% sesaminol and episesaminol, and 60.4% lignan compounds) have revealed the blood cholesterol and triglyceride lowering actions (Japanese Patent No. 3001589) and the action of improving hepatic functions (Japanese Patent No. 3075358). Experiments with refined sesamin/episesamin compositions, say, at a sesamin to episesamin ratio of about 6:4 have revealed the action of improving hepatic functions (61.5% sesamin and 38.0% episesamin) (Japanese Patent No. 3075358), the action of scavenging active oxygen (sesamin:episesamin = ca. 6:4) (JP 6-227977 A), the action of preventing sickness from drinking (55.2% sesamin and 44.4% episesamin; 99.6% purity) (Japanese Patent No. 3124062), and the action of preventing or alleviating symptoms of allergy (55.2% sesamin and 44.4% episesamin; 99.6% purity) (Japanese Patent No. 3512196) and the like; experiments with a sesamin to episesamin ratio of about 1:1 have revealed the action of adjusting the balance between omega 6 and omega 3 unsaturated fatty acids (51.3% sesamin and 47.8% episesamin) (Japanese Patent No. 3512196), the action of suppressing the generation of lipid peroxide (51.3% sesamin and 47.8% episesamin) (JP 5-051388 A), the action of suppressing breast cancer (51.3% sesamin and 47.8% episesamin) (JP 5-43458 A), antihypertensive action (51.5% sesamin, 47.8% episesamin and 1.1% other dioxabicyclo[3,3,0]octane derivative) (JP 8-268887 A), the action of reducing body fat (sesamin:episesamin = ca. 1:1; 99.5% purity) (Japanese Patent No. 3205315), and the action of suppressing prostatomegaly by combination with saw palmetto (sesamin:episesamin = ca. 1:1) (JP 2000-256204 A) and so on.

Recent studies have also revealed the differences between the physiological activities of sesamin and episesamin. For example, rats administered orally with a mixture of sesamin and episesamin (ca. 1:1) were shown to have such an in vivo distribution that the transfer of episesamin into organs was at least twice as much as that of sesamin (Sawada, R. et al., Lipids, 34, 633 (1999)). In addition, experiments where rats were separately administered orally with sesamin and episesamin yielded a report showing that episesamin markedly increased the gene expression and enzymatic activity of β-oxidation enzymes in the liver as compared with sesamin and that there was no difference between sesamin and episesamin in terms of activity for inhibiting fatty acid synthases (Kushiro, M. et al., J. Nutr. Biochem., 13, 289-295 (2002)).

### DISCLOSURE OF THE INVENTION

### PROBLEMS TO BE SOLVED BY THE INVENTION

As described above, sesamins have a variety of physiological activities but they are precious compounds contained in natural sesame seeds in only about 0.1 to 0.5%. The Applicant therefore made a study to develop a method of enhancing the actions of sesamins in order to ensure that they would exhibit their physiological activities in an efficient way. As a result, the Applicant found that α-tocopherol was capable of enhancing the actions of dioxabicyclo[3,3,0]octane derivatives of sesamins (Japanese Patent No. 3283274). However, no method has ever been known that can enhance the physiological activities of sesamins themselves.

### MEANS FOR SOLVING THE PROBLEMS

If the physiological activities of sesamins themselves could be enhanced, the useful physiological activities of sesamins would be exhibited in a more efficient and effective way. Based on this assumption, the present inventors made an intensive study in order to develop a method for enhancing the physiological activities of sesamins themselves.
To begin with, since episesamin was shown to markedly increase the gene expression and enzymatic activity of β-oxidation enzymes in the liver as compared with sesamin (Kushiro, M. et al., J. Nutr. Biochem., 13, 289-295 (2002)), the inventors thought that at least for the action of improving hepatic functions, episesamin would have activity comparable to or greater than sesamin and they made a study to develop a method that allowed episesamin to be efficiently absorbed by the body, thereby enhancing the physiological activities of sesamins. Specifically, sesamin and episesamin were mixed at varying weight ratios to prepare sesamin/episesamin compositions (oil-soluble samples), which were administered perorally to evaluate subsequent oral absorption of episesamin and its transfer into blood circulation. According to the teachings of Sawada, R. et al., Lipids, 34, 633 (1999) and Kushiro, M. et al., J. Nutr. Biochem., 13, 289-295 (2002), the transfer of episesamin into blood circulation was anticipated to be the highest when it was administered independently. Surprisingly, however, contrary to this prediction, sesamin/episesamin compositions containing certain amounts of sesamin allowed episesamin to be absorbed perorally and/or transferred into blood circulation more efficiently than when episesamin was administered alone. The inventors then evaluated the antihypertensive action of sesamins as one of their physiological activities and the gene expression of β-oxidation enzymes in the liver; again surprisingly, sesamin/episesamin compositions containing sesamin and episesamin in specified proportions (from 50:50 to 1:99) were shown to have higher activity than sesamin and episesamin that were administered independently. The present invention has been accomplished on the basis of these findings.

### BRIEF DESCRIPTION OF THE INVENTION

Fig. 1 is a bar graph showing the percent oral absorption of episesamin from orally administered mixtures of sesamin and episesamin at varying compositional ratios of 4:6, 5:5, 3:7, 2:8, 1:9, and 0:10;
Fig. 2 is a bar graph showing the percent oral absorption of episesamin from orally administered mixtures of sesamin and episesamin at varying compositional ratios of 1:9, 0.5:9.5, and 0:10;
Fig. 3 is a graph showing the 24-hr blood pressure changes in DOCA-salt hypertensive rats which received a single administration of mixtures of sesamin and episesamin at varying compositional ratios of 10:0, 5:5, and 0:10;
Fig. 4 is a graph showing the 8-hr blood pressure changes that occurred in DOCA-salt hypertensive rats at 8 hours after they were administered with mixtures of sesamin and episesamin at varying compositional ratios of 10:0, 5:5, and 0:10;
Fig. 5 is a graph showing the blood pressure changes that occurred in DOCA-salt hypertensive rats at 8 hours after they were administered with mixtures of sesamin and episesamin at varying compositional ratios of 5:5, 3:7, and 1:9; and
Figs. 6A and 6B are graphs showing how mixtures of sesamin and episesamin administered at varying compositional ratios of 10:0, 5:5, 3:7, 1:9, and 0:10 affected the expression of β-oxidation genes.

### EMBODIMENT FOR CARRYING OUT THE INVENTION

The present invention relates to sesamin/episesamin compositions that allow sesamins to exhibit their physiological activities in an efficient and effective way. The invention also relates to a method of enhancing the physiological activities of sesamins by using the compositions. Specifically, the invention relates to the following:
1. A sesamin/episesamin composition comprising sesamin and episesamin in proportions of 50:50 to 1:99 by weight ratio.
2. The sesamin/episesamin composition as defined above in 1 which comprises sesamin and episesamin in proportions of 39:61 to 1:99 by weight ratio.
3. The sesamin/episesamin composition as defined above in 1 which comprises sesamin and episesamin in proportions of 30:70 to 5:95 by weight ratio.
4. The sesamin/episesamin composition as defined above in any one of 1-3, wherein the total amount of sesamin and episesamin is at least 51 wt% of the composition.
5. Food or beverage containing the sesamin/episesamin composition as defined above in 1-4.
6. A pharmaceutical composition containing the sesamin/episesamin composition as defined above in 1-4.
7. Animal feed and pet food containing the sesamin/episesamin composition as defined above in 1-4.
8. A method of enhancing a physiological activity of sesamins by the sesamin/episesamin compostion as defined above in 1-4.
9. The method as defined above in 8, wherein the physiological activity of sesamins is a blood cholesterol lowering action, a triglyceride lowering action, an antihypertensive action, an action for antioxidation of unsaturated fatty acids, a hepatic function improving action, an active oxygen scavenging action, an action for inhibiting Δ5 unsaturation enzyme, or an action for in vivo stabilization of highly unsaturated fatty acids.

The sesamin/episesamin compositions of the present invention are characterized by containing sesamin and episesamin in specified proportions. The proportions of sesamin and episesamin range preferably from 50:50 to 1:99, more preferably from 39:61 to 1:99, even more preferably from 30:70 to 5:95 by weight ratio. The sesamin/episesamin compositions of the present invention which contain sesamin and episesamin in the above specified proportions have such a nature that they allow episesamin to be perorally absorbed and/or transferred into blood circulation more efficiently than when it is administered alone. In addition, the sesamin/episesamin compositions of the present invention which contain sesamin and episesamin in the above specified proportions are characterized in that the physiological activities of sesamins, in particular, the antihypertensive action, hepatic function improving action and the lipid metabolism improving action are enhanced in comparison with the case where sesamin or episesamin is administered independently and, hence, sesamins are allowed to exhibit the physiological activities of their own in an efficient and effective way.

The methods of producing the sesamin/episesamin compositions of the present invention are not limited in any particular way and, in one example, the compositions of the present invention can be produced by incorporating refined forms of sesamin and episesamin in desired proportions. The refined forms of sesamin and episesamin can, for example, be prepared by the method described in Fukuda, Y. et al., J. Am. Oil Chem. Soc., 63, 1027-1031 (1986) and details of the method are also described in Example 1 to be given later. However, the refined forms of sesamin and episesamin may be obtained by any other suitable methods.

Another embodiment of the present invention comprises preparing a feed mixture of sesamin and episesamin, determining the weight ratio of the two components, and adding a refined form of sesamin or episesamin to the feed mixture until a sesamin-episesamin compositional ratio in the range specified by the present invention is reached. Alternatively, a mixture of sesamin and episesamin which is richer in one component than the other may be added to the feed mixture.

Yet another embodiment of the present invention comprises removing sesamin from a mixture of sesamin and episesamin (which may have a compositional ratio of 1:1). Sesamin can be removed by recrystallization, chromatography, distillation and other techniques that may be employed either independently or as combined appropriately.

In a further embodiment, the present invention may be implemented by applying epimerization reaction to sesamin alone, a sesamin-episesamin mixture or episesamin alone so as to adjust the sesamin-episesamin compositional ratio.

The present inventors have recently developed the following method with a view to enhancing the concentration of episesamin in a sesamin-episesamin mixture and this method can also be applied: the sesamin-episesamin mixture is dissolved in alcohol or an aqueous solution of alcohol; then, episesamin is selectively crystallized by recrystallization so as to give a sesamin-episesamin mixture with an enhanced episesamin concentration.

Alternatively, a sesamin-episesamin mixture is dissolved in a specified oil or fat under heating and then episesamin is selectively crystallized by recrystallization so as to give a sesamin-episesamin mixture with an enhanced episesamin concentration.

In yet another method that can be employed, a sesamin-episesamin mixture is dissolved in water, a watersoluble solvent or a mixture thereof and after optional addition of an alkali, sesamins are caused to precipitate, thereby giving a sesamin-episesamin mixture with an enhanced episesamin concentration.

In implementing the present invention, the sesamin-episesamin compositional ratios of the feed mixture and the compositions of the present invention can be determined by high-performance liquid chromatography (HPLC) and any other known methods of analysis.

The above-mentioned feed mixture comprising sesamin and episesamin may be exemplified by sesame lignan compositions refined from sesame seed, sesame cake or meal, and sesame oil. Sesame lignan compositions may be purchased commercial products (as from Takemoto Oil & Fat Co., Ltd.) but they can also be obtained from sesame oil by one of many known methods including the one described in Fukuda, Y. et al., J. Am. Oil Chem. Soc., 63, 1027-1031 (1986) and a method comprising the steps of distilling sesame oil by steam to give a distillate and separating sesamins as crystallized from the distillate in the presence of an alkali (see JP 10-7676 A). It should be noted here that the feed mixture comprising sesamin and episesamin is by no means limited to the above-mentioned sesame lignan compositions as refined from sesame seed, sesame cake or meal, and sesame oil; they may be replaced by what substantially contains the two ingredients, as exemplified by Acanthopanax trifoliate root bark, paulownia tree, and ginkgo bark, etc.

The sesamin/episesamin compositions of the present invention have such a nature that they allow episesamin to be perorally absorbed and/or transferred into blood circulation in an efficient way. The oral absorption of episesamin and its transfer into blood circulation can be verified by, for example, the method described in Example 2 to be given later (quantification by LC-MS/MS) but other suitable methods may be employed.

Since the sesamin/episesamin compositions of the present invention have such a nature that they allow episesamin to be perorally absorbed and/or transferred into blood circulation more efficiently, they enable episesamin to exhibit its various actions in an efficient and effective way. Actions of episesamin include, for example, improvement in the transfer of sesamins into organs and improvement in hepatic functions.

Although details of mechanism are not known, the sesamin/episesamin compositions of the present invention have such a nature that the physiological activities of sesamins are enhanced in comparison with the case where sesamin or episesamin is administered independently and, hence, they enable sesamins to exhibit the various physiological activities of their own in an efficient and effective way. The physiological activities of sesamins include a blood cholesterol and/or triglyceride lowering action, an antihypertensive action, an action for antioxidation of unsaturated fatty acids, a hepatic function improving action, an active oxygen scavenging action, an action for inhibiting Δ5 unsaturation enzyme, an action for in vivo stabilization of highly unsaturated fatty acids, etc.

The sesamin/episesamin composition of the present invention can advantageously be used in the form of food or beverage or a pharmaceutical composition. The proportion of the sesamin/episesamin composition of the present invention that may be added to food or beverage or a pharmaceutical composition is not limited to any particular value as long as its functions are retained but an appropriate value can usually be selected from the range of 0.01-100 wt%. Note that the content of episesamin to be contained in the food or beverage or the pharmaceutical composition is between 0.5 and 100 mg, preferably between 1 and 60 mg, more preferably between 3 and 60 mg, per oral intake.

To produce the food or beverage or pharmaceutical composition that contain the sesamin/episesamin composition of the present invention, the latter may be added to food or beverage or pharmaceutical composition. Alternatively, sesamin, episesamin and/or a sesamin-episesamin mixture may be added either simultaneously or separately in such a way that sesamin and episesamin are contained at weight ratios in the range specified by the present invention. The weight ratio of the two compounds may be adjusted by appropriately applying the methods described in connection with the production of the sesamin/episesamin composition.

The food or beverage or pharmaceutical composition that contain the sesamin/episesamin composition of the present invention is not limited to any particular form and they can be prepared in any desired forms including solids such as powder, granules and tablets, solutions such as liquids and emulsions, capsules filled with such compositions in solution, and semi-solids such as pastes.

In this case, the sesamin/episesamin composition of the present invention may be combined with a diluent, a carrier or other additives and any desired formulating procedure may be applied to make preparations. The diluent or carrier that can be employed is not limited in any particular way as long as it does not interfere with the physiological activities of sesamins and examples include saccharides such as sucrose, glucose, fructose, maltose, trehalose, lactose, oligosaccharides, dextrin, dextran, cyclodextrin, starch, syrup and isomerized liquid sugar, alcohols such as ethanol, propylene glycol and glycerin, sugar alcohols such as sorbitol, mannitol, erythritol, lactitol, xylitol, maltitol, reduced palatinose and reduced starch decomposition product, solvents such as triacetin, polysaccharides such as gum arabic, carrageenan, xanthan gum, guar gum, gellan gum and pectin, as well as water. Examples of additives include aids such as chelatants, flavors, spice extracts, and antiseptics, etc.

For reasons such as convenience in use, the making of the above-mentioned preparation using the diluent, carrier or additive described above is desirably formulated in such a way that the sesamin/episesamin composition is contained in a proportion of 0.01-100 wt%, preferably 0.1-50 wt%, in 100 wt% of the preparation. In this case, if the sesamin/episesamin composition as the feed contains sesamin and episesamin in a total amount of less than 51% of the composition, the content of sesamins is unduly low and if a preferred amount of sesamins is incorporated, the volume of the resulting preparation containing the sesamin/episesamin composition becomes so large that inconvenience in intake may often result. In particular, in the case where the sesamin/episesamin composition of the present invention is dissolved in fat or oil and subjected to a formulating procedure to make a preparation for oral administration such as a soft capsule, a need arises to increase the size of the preparation or ingest many units of the preparation at a time, causing not only inconvenience in intake but also the problem of ingesting a more-than-necessary amount of fat or oil that serves as a solvent. Therefore, from the viewpoint of taking in a smaller amount, too, it is preferred to use a sesamin/episesamin composition that has been prepared in such a way that the total amount of sesamin and episesamin accounts for at least 51% of the composition, and it is more preferred that the sesamin/episesamin composition has a purity of at least 90%.

Examples of the sesamin/episesamin composition containing foods and beverages include foods and beverages such as supplements that contain the sesamin/episesamin composition of the present invention per se, as well as functional foods with health-promoting benefits [inclusive of foods for specified health use (FOSHU) and qualified FOSHU] that have the sesamin/episesamin composition of the present invention incorporated as an ingredient in ordinary foods and beverages so that the latter are provided with the physiological activities of sesamins. These foods and beverages also include those which are characterized by having sesamin and episesamin contained or added at weight ratios in the range specified by the present invention and which are put in containers or accompanied by descriptions, both referring to those foods or beverages having a blood cholesterol and/or triglyceride lowering action and other physiological actions. The other physiological actions that can be indicated for the foods and beverages include an antihypertensive action, an action for antioxidation of unsaturated fatty acids, a hepatic function improving action, an active oxygen scavenging action, an action for inhibiting Δ5 unsaturation enzyme, an action for in vivo stabilization of highly unsaturated fatty acids, etc.

The foods and beverages that may contain the sesamin/episesamin composition of the present invention are not limited in any particular way but include fruit juice containing drinks, soft drinks, sports drinks, alcoholic beverages, drinks such as tea, agricultural foods such as bread, noodles, cooked rice, confectionery (biscuits, cakes, candies, chocolates, and Japanese sweets), soybean cake and products processed therefrom, *sake,* medicated liquor, fermented foods such as *mirin,* edible vinegar, soya sauce and *miso,* oil- and fat-based foods such as salad dressing, mayonnaise, butter, margarine, shortening, and edible fat and oil, processed meats such as ham, bacon and sausage, dairy products such as yoghurt, aquatic foods such as *kamaboko, ageten, hanpen,* etc., animal feed, and pet food.

### [Examples]

On the following pages, the present invention is described in greater detail by reference to the following examples, which are by no means intended to limit the present invention.

### Example 1: Refining of Sesamin and Episesamin

The sesamin-episesamin mixture (a feed mixture comprising sesamin and episesamin) used in this example was purchased from Takemoto Oil & Fat Co., Ltd. as a mixture having a 99.5% purity in terms of sesamins and containing sesamin and episesamin in proportions of 51.8:48.0 by weight percent.

The high purity refined forms of sesamin and episesamin were prepared using reverse-phase HPLC under the following conditions. To be more specific, 100 mg of the above-mentioned commercial product of Takemoto Oil & Fat Co., Ltd. as dissolved in DMSO was injected into a column of Develosil ODS-UG-5 (20^{φ} x 250 mm; product of Nomura Chemical Co., Ltd.) and eluted by a 20% → 80% acetonitrile's linear gradient (elution time, 100 min; flow rate, 5 ml/min); the absorbance at 280 nm was monitored for fractionation; as a result, sesamin (retention time, 89 min) was recovered in 38.9 mg and episesamin (retention time, 94 min) in 35.0 mg. This procedure was performed 10 times and the thus obtained refined specimens (each having a purity of at least 99%) were subjected to testing in Example 2.

### Example 2: Sesamin-Episesamin Compositional Ratio and Oral Absorption of Episesamin and Its Transfer into Blood Circulation (I)

SD (IGS) male rats (6-wk old) were purchased from CHARLES RIVER JAPAN INC. and habituated in the test environment for a week; the animals that were seen to grow normally were subjected to the test. The rats fasted overnight were divided into six groups each consisting of 5 heads; the high purity sesamin and episesamin obtained in Example 1 were incorporated at varying weight ratios of 6:4, 5:5, 4:6, 3:7, 1:9 and 0:10 to prepare sesamin/episesamin mixtures, which were dissolved in olive oil at a dose of 10 mg/kg and administered perorally to the rats via a feeding tube. Both before the administration and 1, 2, 4, 6, 8 and 24 hours after the administration, blood was sampled from the tail vein of each animal into a heparinized collecting tube and centrifuged (8,000 rpm x 10 min) to prepare plasma samples. After adding an internal standard, solid-phase extraction was performed with Oassis HLB and the liquid extract was concentrated under vacuum, suspended in methanol, passed through a filter, and subjected to LC-MS/MS for quantification of sesamin and episesamin. The quantity of sesamin or episesamin was determined from the ratio between the peak area of sesamin or episesamin and that of UDESMIN (Funakoshi Co, Ltd.) used as the internal standard. The conditions of LC-MS/MS analysis are shown below.

### (HPLC)

Column: Develosil C30-UG-5 (5 µm, 2.0^{φ}x 50 mm; product of Nomura Chemical Co., Ltd.)
Mobile phases: A, distilled water; B, methanol; D, aqueous solution of 100 mM ammonium acetate
Flow rate: 0.25 mL/min
Gradient: Linear gradient consisting of 2 min with 55% B and 10% D, then 3 min with B increasing from 55% to 60% but D remaining at 10%, and finally 2 min with B further increasing from 60% to 85% but D remaining at 10%

### (MS/MS)

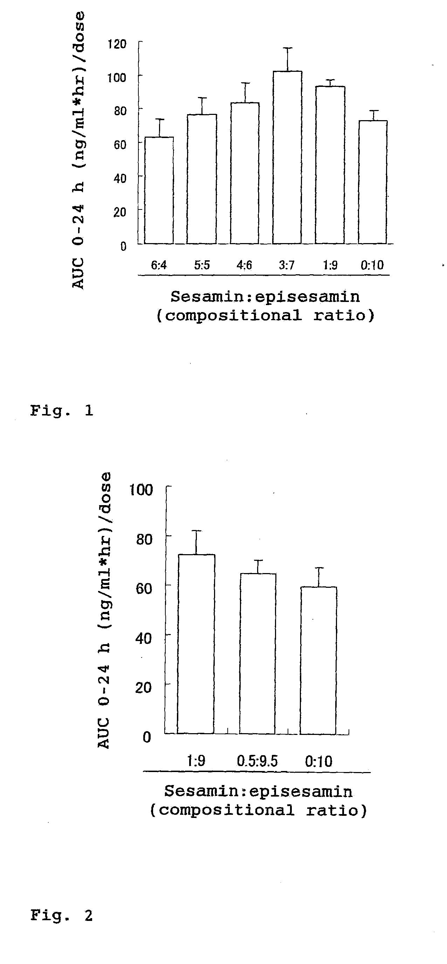

Measuring mode: Selective reaction monitoring
Detection: Episesamin (retention time, ca. 5.6 min);
precursor ion m/z = 372 ([M+NH₄]⁺), product ion m/z = 233 UDESMIN (retention time, ca. 2.8 min); precursor
ion m/z = 404 ([M+NH₄]⁺), product ion m/z = 249
Ionization method: ESI method
The concentration of episesamin in blood peaked in about 6 hours after the administration, then decreased gradually until it became almost undetectable in 24 hours. From the blood concentration curve for 0-24 hours, the area under the curve (AUC) was determined as an index of absorbed quantity. Since different animal groups were administered different amounts of episesamin, it is appropriate to make comparison in terms of percent absorption as corrected by dividing each value of AUC by the relative amount of episesamin administered. The results are shown in Fig. 1. When episesamin was administered alone, its absorption was 72.5%; on the other hand, when sesamin and episesamin were administered at a ratio of 50:50, the absorption of episesamin increased to a higher value of 76.7%. The percent absorption of episesamin further improved as its relative weight was increased and the highest value was reached at a sesamin to episesamin ratio of 30:70; a sufficiently high value was shown event at a ratio of 10:90. Thus, it became clear that when sesamin and episesamin were incorporated at compositional ratios between 50:50 and 10:90, the percent absorption of episesamin was higher than when it was administered alone (sesamin to episesamin weight ratio of 0:100).

Therefore, the results of Example 2 show that the percent absorption of episesamin is improved by incorporating sesamin and episesamin at specified weight ratios.

### Example 3: Sesamin-Episesamin Compositional Ratio and Oral Absorption of Episesamin and Its Transfer into Blood Circulation (II)

With a view to prescribing effective mixing ratios of sesamin, the action of further lowered ratios of sesamin was evaluated in accordance with the method of Example 2. To be more specific, the high purity sesamin and episesamin obtained in Example 1 were incorporated at varying weight ratios of 1:9, 0.5:9.5 and 0:10 to prepare sesamin/episesamin mixtures, which were dissolved in olive oil at a dose of 10 mg/kg and administered perorally to the rats via a feeding tube. The percent absorption of episesamin was calculated and the results are shown in Fig. 2. When episesamin was administered alone, its absorption was 59.7%; on the other hand, when sesamin and episesamin were administered at ratios of 5:95 and 10:90, the absorption of episesamin increased to higher values of 64.7% and 72.4%, respectively. Thus, it became clear that even when sesamin and episesamin were incorporated at a compositional ratio of 5:95, the percent absorption of episesamin was higher than when it was administered alone (sesamin to episesamin weight ratio of 0:100).

### Example 4: Blood Pressure Lowering Action in Deoxycorticosterone Acetate-Salt (DOCA-Salt) Induced Hypertension Model Rats

SD male rats (6-wk old) (purchased from Japan SLC, Inc.) were subjected to surgery for removing the right kidney under anesthetization with Nembutal. After recuperation for a week, the rats were administered subcutaneously with DOCA-salt twice a week at a dose of 15 mg/kg, and allowed to drink 1% saline. The systolic blood pressure (SBP) of each animal was measured by a tail-cuff method with a non-invasive automatic blood pressure measuring apparatus (BP-98A, Softron, Tokyo). At 5 weeks after the start of treatment with DOCA-salt, blood pressure was measured and the animals in which adequate elevation of blood pressure was verified were used in the following experiment. Rats fasted for 8 hours were divided into a control group and three test groups. The control group was administered olive oil only. For the test groups, the high purity sesamin and episesamin obtained in Example 1 were incorporated at varying weight ratios of 10:0, 5:5 and 0:10 to prepare sesamin/episesamin mixtures, which were suspended in olive oil at a dose of 100 mg/kg. Single oral administration of olive oil or the suspension was effected via a feeding tube. Both before the administration and 2, 4, 6, 8, 10, 12 and 24 hours after the administration, SBP measurement was conducted. Diet was applied after the blood pressure measurement at 12 hours. Testing was conducted at 1-wk intervals and the same head was subjected to no more than three measurements.

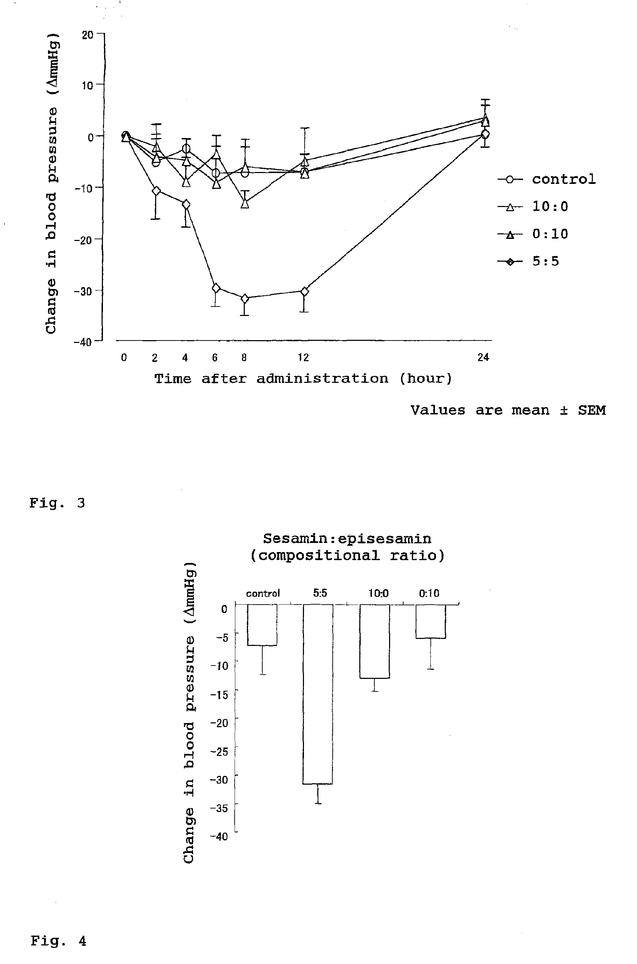

The changes in blood pressure (n=5-7) for 24 hours after the single administration are shown in Fig. 3. No significant changes were observed in the groups solely administered with sesamin, episesamin, and olive oil, respectively. On the other hand, in the group administered with the sesamin/episesamin (5:5) mixture, a marked blood pressure lowering action was observed, with a peak occurring 8 hours after the administration. A comparison of the changes in blood pressure that occurred 8 hours after the administration showed that the group administered with the sesamin/episesamin (5:5) mixture had a blood pressure of -32 mmHg which was obviously lower than the value in the sesamin group (-13 mmHg) and that in the episesamin group (-6 mmHg) (see Fig. 4).

### Example 5: Effects of Mixing Ratio on the Blood Pressure Lowing Action in DOCA-salt Induced Hypertension Model Rats

With the most potent sesamin/episesamin (5:5) mixture used as the reference, a comparative test was conducted in accordance with the method of Example 4 to know how the difference in mixing ratio would affect the effectiveness. Since very high potency was observed at 100 mg/kg, the dose was lowered by one half in order to increase the visibility of the difference.

Sesamin and episesamin were incorporated at varying weight ratios of 5:5, 3:7 and 1:9 to prepare sesamin/episesamin mixtures, which were dissolved in olive oil at a dose of 50 mg/kg. Single oral administration of the solution was effected via a feeding tube, and for blood pressure measurement was conducted.

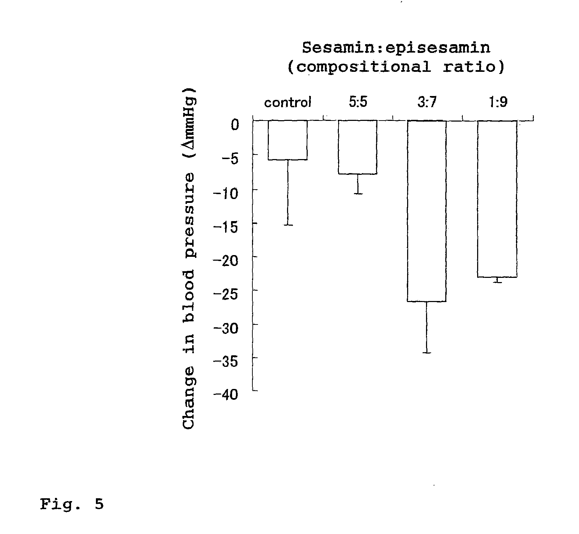

The changes in blood pressure (n=3) at 8 hours after the administration are shown in Fig. 5. The sesamin/episesamin (5:5) mixture was found to have no blood pressure lowering action at the dose of 50 mg/kg. On the other hand, the sesamin/episesamin (3:7) mixture and the (1:9) mixture were found to have a blood pressure lowering action which proved to be particularly high at 3:7.

These results show that mixing sesamin and episesamin at specified ratios is effective in lowering the blood pressure synergistically. The effectiveness was found to be the strongest at the mixing ratio of 3:7.

### Example 6: Effects on the Expression of β-Oxidation Genes

Wistar male rats (6-wk old) were purchased from CLEA Japan, Inc. and habituated in the test environment for a week; the animals that were seen to grow normally were subjected to the test. The rats were divided into six groups each consisting of 5 heads. The control group was administered with olive oil only. For the test groups, the high purity sesamin and episesamin obtained in Example 1 were incorporated at varying weight ratios of 10:0, 5:5, 3:7, 1:9 and 0:10 to prepare sesamin/episesamin mixtures, which were suspended in olive oil at a dose of 200 mg/kg. The rats were administered perorally with olive oil or the suspension three times at 24-hr intervals. Four hours after the final administration, the kidney was removed from each rat under anesthetization with Nembutal; after extracting RNA, effects on the expression of 10 β-oxidation genes were investigated by quantitative RT-PCR using TaqMan chemistry (Heid CA, Stevens J, Livak KJ, Williams PM, Real time quantitative PCR, Genome Res. 1996 6(10):986-94).

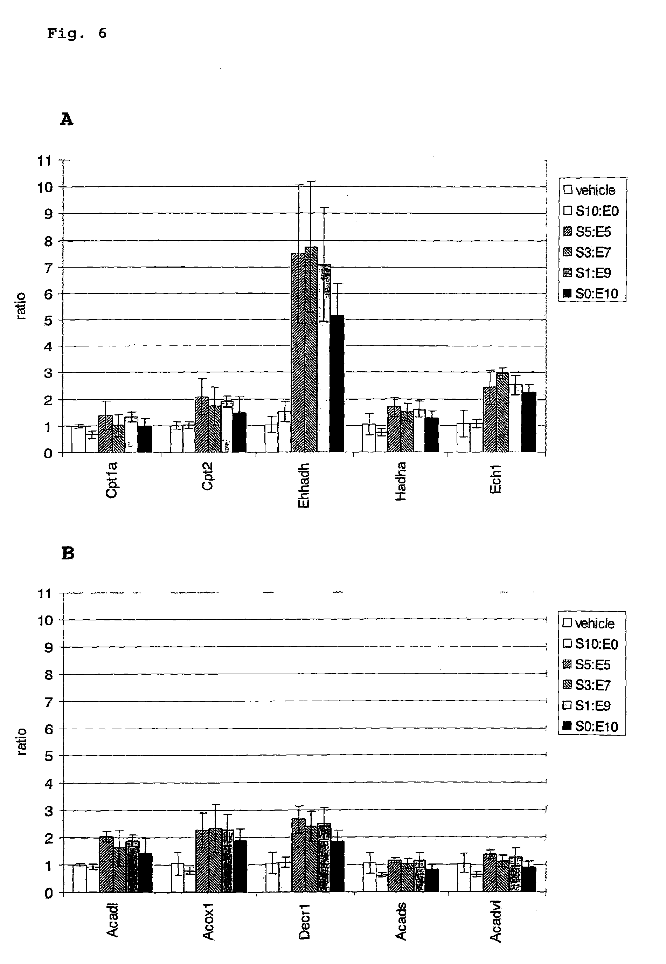

The genes evaluated are listed in the following Table 1 and the amounts of gene expression are shown in Figs. 6A and 6B. Under the conditions employed, the expression of β-oxidation genes hardly rose in the group administered with sesamin alone whereas many genes were expressed in increased amounts in the group administered with episesamin alone. In contrast, each of the groups administered with the sesamin/episesamin mixture experienced more elevated gene expression than the group administered with episesamin alone. The effect of the mixing ratio was somewhat variable from one gene to another but no significant difference was found among the three groups administered with the sesamin/episesamin mixture.

**Table 1. List of Genes Evaluated**

| Enzyme | Gene Name | Gene symbol |
|---|---|---|
| carnitine palmitoyltransferase | carnitine palmitoyltransferase 1, liver | Cpt1a |
| | carnitine palmitoyltransferase 2 | Cpt2 |
| bifunctional enzyme | enoyl-Coenzyme A, hydratase/3-hydroxyacyl Coenzyme A dehydrogenase | Ehhadh |
| trifunctional enzyme | hydroxyacyl-Coenzyme A dehydrogenase/3-ketoacyl-Coenzyme A thiolase/enoyl-Coenzyme A hydratase (trifunctional protein), alpha ubunit | Hadha |
| enoyl-CoA hydratase | enoyl coenzyme A hydratase 1, peroxisomal | Ech1 |
| acetyl-Coenzyme A dehydrogenase | acetyl-Coenzyme A dehydrogenase, long-chain | Acadl |
| acyl-CoA oxidase | acyl-Coenzyme A oxidase 1, palmitoyl | Acox1 |
| 2,4-dienoyl CoA reductase | 2,4-dienoyl CoA reductase 1, mitochondrial | Decr1 |
| acyl-coenzyme A dehydrogenase | acyl-coenzyme A dehydrogenase, short chain | Acads |
| | acyl-Coenzyme A dehydrogenase, very long chain | Acadvl |

The above results show that mixing episesamin with sesamin is effective in enhancing the expression of β-oxidation genes synergistically.

### [Examples of Preparations]

Examples of preparations are shown below. The term "mixture" means a sesamin/episesamin composition having a sesamin/episesamin compositional ratio within the range specified by the present invention (sesamin: episesamin = 30:70; 99.5% purity).

(Preparation 1) Butter

| | |
|---|---|
| "Mixture" | 1.2 g |
| Butterfat | 100 g |
| Tocopherol acetate | 1.2 g |

A hundred grams of butterfat separated from buttermilk by the churning step in the process of butter making was mixed with 1.2 g of the mixture and 1.2 g of tocopherol acetate, followed by working to give a uniform texture of butter containing the composition of the present invention.

(Preparation 2) Granule

| | |
|---|---|
| "Mixture" | 0.25 g |
| Tocopherol acetate | 0.25 g |
| Silicic acid anhydride | 20.5 g |
| Corn starch | 79 g |

After uniformly mixing the powder consisting of the above ingredients, 100 ml of 10% hydroxypropylcellulose in ethanol was added and a usual procedure consisting of blending, extruding and drying was followed to make granules.

(Preparation 3) Tablet

| | |
|---|---|
| "Mixture" | 3.5 g |
| Tocopherol acetate | 0.5 g |
| Silicic acid anhydride | 20 g |
| Microcrystalline cellulose | 10 g |
| Magnesium stearate | 3 g |
| Lactose | 60 g |

These ingredients were mixed and tableted with a single-action tableting machine to make tablets each having a diameter of 7 mm and weighing 100 mg.

(Preparation 4) Capsule

| | |
|---|---|
| Gelatin | 70.0% |
| Glycerin | 22.9% |
| Methyl paraoxybenzoate | 0.15% |
| Propyl paraoxybenzoate | 0.51% |
| Water | q.s. |
| Total | 100% |

A soft capsule shell made of these ingredients was filled with the following composition by the usual method to make soft capsules each weighing 200 mg.

| | |
|---|---|
| "Mixture" | 10.8 mg |
| Wheat beeswax | 30 mg |
| α-Tocopherol | 20 mg |
| Palm oil | 10 mg |
| Wheat germ oil | q.s. |
| Total | 100% |

(Preparation 5) Drink

| | |
|---|---|
| Major taste givers: sodium DL-tartrate | 0.1 g |
| succinic acid | 0.009 g |
| Sweetener: liquid sugar | 800 g |
| Acidifier: citric acid | 12 g |
| Vitamin: vitamin C | 10 g |
| "Mixture" | 1 g |
| Vitamin E | 30 g |
| Cyclodextrin | 5 g |
| Flavor | 15 ml |
| Potassium chloride | 1 g |
| Magnesium sulfate | 0.5 g |

These ingredients were mixed together and water was added to make a volume of 10 liters. The resulting preparations would be drinkable in an amount of about 100 ml at a time.

### EFFECTS OF THE INVENTION

By ingesting the sesamin/episesamin compositions of the present invention, the various physiological activities of sesamins can be exhibited efficiently. In particular, the sesamin/episesamin compositions of the present invention are high in oral absorption of episesamin and its transfer into blood circulation, so they enable episesamin to exhibit its various physiological activities efficiently. In addition, by mixing episesamin with sesamin at specified ratios, the blood lowering action of sesamins and the expression of β-oxidation genes can be enhanced synergistically. However, sesamin and episesamin are both of plant origin, so they have mild actions and feature extremely high safety. Therefore, the compositions of the present invention are very useful and the scope of their application covers not only health foods but they are also applicable to pharmaceuticals.

## Claims

1. A sesamin/episesamin composition comprising sesamin and episesamin in proportions of 50:50 to 1:99 by weight ratio.

2. The sesamin/episesamin composition according to claim 1 which comprises sesamin and episesamin in proportions of 39:61 to 1:99 by weight ratio.

3. The sesamin/episesamin composition according to claim 1 which comprises sesamin and episesamin in proportions of 30:70 to 5:95 by weight ratio.

4. The sesamin/episesamin composition according to any one of claims 1-3, wherein the total amount of sesamin and episesamin is at least 51 wt% of the composition.

5. Food or beverage containing the sesamin/episesamin composition according to claims 1-4.

6. A pharmaceutical composition containing the sesamin/episesamin composition according to claims 1-4.

7. Animal feed and pet food containing the sesamin/episesamin composition according to claims 1-4.

8. A method of enhancing a physiological activity of sesamins by the sesamin/episesamin composition according to claims 1-4.

9. The method according to claim 8, wherein the physiological activity of sesamins is a blood cholesterol lowering action, a triglyceride lowering action, an antihypertensive action, an action for antioxidation of unsaturated fatty acids, a hepatic function improving action, an active oxygen scavenging action, an action for inhibiting Δ5 unsaturation enzyme, or an action for in vivo stabilization of highly unsaturated fatty acids.
